Europäisches Patentamt

European Patent Office

Office européen des brevets

(19)

(11) Publication number: **0 209 539 B1**

# EUROPEAN PATENT SPECIFICATION

(12)

(45) Date of publication of patent specification: **20.05.92**

(51) Int. Cl.⁵: **C07K 7/10**, C07K 13/00, A61K 37/24

(21) Application number: **86900400.2**

(22) Date of filing: **27.11.85**

(86) International application number:
**PCT/US85/02358**

(87) International publication number:
**WO 86/04068 (17.07.86 86/17)**

The file contains technical information submitted after the application was filed and not included in this specification

(54) **HOMOGENEOUS ERYTHROPOIETIN.**

(30) Priority: **11.01.85 US 690853**

(43) Date of publication of application:
**28.01.87 Bulletin 87/05**

(45) Publication of the grant of the patent:
**20.05.92 Bulletin 92/21**

(84) Designated Contracting States:
**AT BE CH DE FR GB IT LI LU NL SE**

(56) References cited:
**EP-A- 0 148 605      WO-A-86/03520
US-A- 4 254 095      US-A- 4 289 690
US-A- 4 377 482      US-A- 4 377 513
US-A- 4 465 624      US-A- 4 558 005
US-A- 4 558 006**

(73) Proprietor: **GENETICS INSTITUTE, INC.
87 Cambridge Park Drive
Cambridge, Massachusetts 02140(US)**

(72) Inventor: **HEWICK, Rodney, M.
16 Woodcliffe Rd
Lexington, MA 01742(US)**
Inventor: **SEEHRA, Jasbir, S.
69 Ridge St.
Arlington, MA 02175(US)**

(74) Representative: **Liska, Horst, Dr.-Ing. et al
Patentanwälte H. Weickmann, Dr. K. Fincke,
F.A. Weickmann, B. Huber, Dr. H. Liska, Dr. J.
Prechtel Kopernikusstrasse 9 Postfach 86 08
20
W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.08/2.19/2.0)

CHEMICAL ABSTRACTS, vol. 103, no. 25, 23rd December 1985, page 135, abstract no. 206714q, Columbus, Ohio, US; R.D. LANGE et al.: "Preparation of purified erythropoietin by high performance liquid chromatography", & BLOOD CELLS 1984, 10(2-3), 305-14

PROC. NATL. ACAD. SCI. USA, vol. 81, May 1984, pages 2708-2712; S. LEE-HUANG: "Cloning and expression of human erythropoietin cDNA in Escherichia coli"

THE JOURNAL OF BIOLOGICAL CHEMISTRY, vol. 259, no. 5, 10th March 1984, pages 2707-2710, The American Society of Biological Chemists, Inc., US; S.-I. YANAGAWA et al.: "Isolation of human erythropoietin with monoclonal antibodies"

NATURE, vol. 313, 28th February 1985, pages 806-810; K. JACOBS et al.: "Isolation and characterization of genomic and cDNA clones of human erythropoietin"

N Miyake et al., J.Biol. Chem.252, No.15, (1977), 5558-5564

N Parsons et al., Endocrinology, 114, No.6, (1984), 2223-2227

Biochem. 28 (1987) 2633-2638

Fed. Proc. Fed Am Soc. Exp. Biol (1983) 42 1872 No.672

Blood Cells (1984) 10 : 304-314

Cold Spring Harbor Symposium1986, pp.693-702

Immunobiol. Vol 172, 213-224 (1986)

Excerpt of the US decision (US District Court, MA)

Decision T 148/87

## Description

Erythrocytes (also called red blood cells) serve a critical role in carrying oxygen to mammalian tissues. They are produced by the maturation and differentiation of erythroblasts in bone marrow. Erythropoietin (also referred to as EPO) is a glycoprotein which naturally stimulates erythrocyte formation in mammals.

In certain clinical states, e.g. various anemias, the level of erythrocytes is undesirably low. Exogenously administered EPO has promise as a therapeutic agent for the clinical treatment of such conditions. For therapeutic use, it is highly desirable that the EPO be homogeneous. Unfortunately, exogenous EPO is not in practical use due to its low availability and heterogeneity.

The preparation heretofore of erythropoietin-related products has generally been via the concentration and purification of urine from patients exhibiting high EPO levels. See, for example, U.S. Patent Nos. 3,865,801; 4,303,650 and 4,397,840. In particular, EPO has been purified from the urine of patients with aplastic anemia by the method described by Miyake et al., J. Biol. Chem., 252:5558 (1977). It had been thought that EPO purified by this method was homogeneous because it moved as a single band during electrophoresis.

We have now discovered that this seemingly homogeneous EPO composition produced by the method of Miyake et al. is composed of several polypeptide components ranging from 30,000 to 70,000 daltons.

The present invention provides for the first time a homogeneous EPO composition. We prepared such a composition by subjecting an erythropoietin solution which has been partially purified by the Miyake-type method to reverse phase high performance liquid chromatography and eluting the EPO protein. We thereby produced homogeneous EPO characterized by

(a) movement as a single peak on reverse phase-HPLC, (b) a molecular weight of about 34,000 daltons on SDS PAGE, and (c) a specific activity of at least 160,000 IU per absorbance unit at 280 nanometers. Specific Activity is formally designated in terms of IU's per ml per absorbance unit at 200 nm. For convenience, the specific activity may be referred to in standard fashion, simply in terms of IU's.

### Brief Description of the Drawings and Table

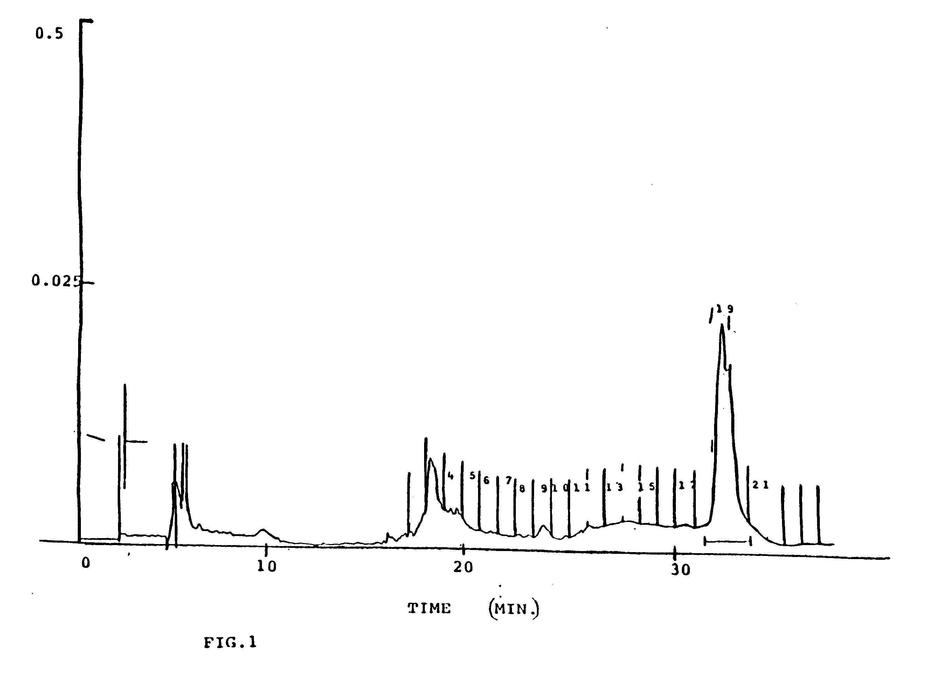

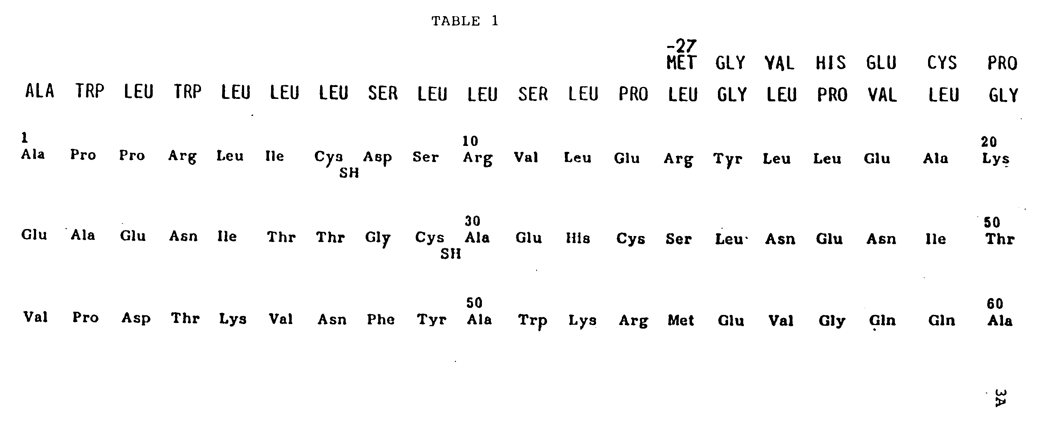

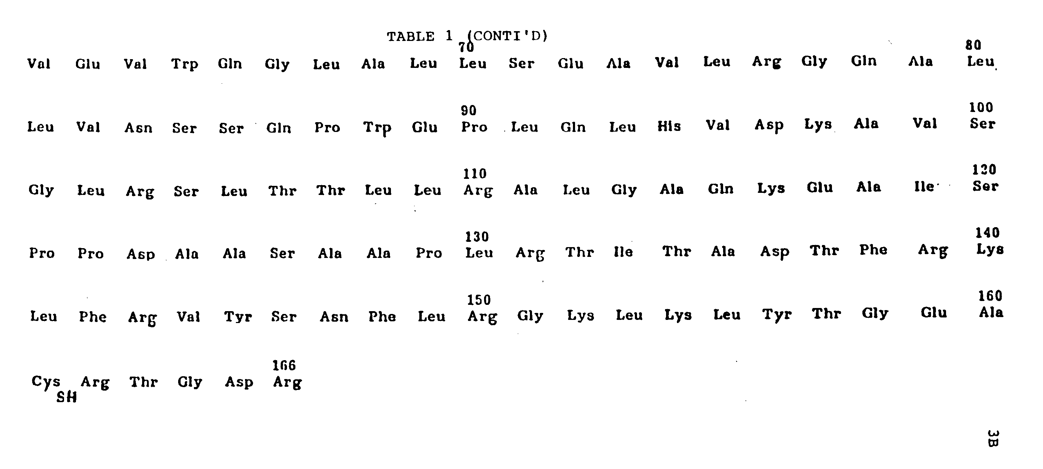

FIG. 1 is an elution profile of an EPO composition treated by reverse phase high performance liquid chromatography in accord with the present invention illustrating absorbance of fractions at 280 nm versus time.

Table 1 is the amino acid sequence of a human EPO protein including its secretory leader sequence.

The availability of homogeneous EPO will permit the sequencing of its amino acid composition by techniques known in the art to permit the construction of probes with which to "fish out" the corresponding human genes and thus facilitate production of EPO by recombinant DNA techniques.

In fact, an operable recombinant DNA process for producing EPO which relies on sequence information obtained through the use of EPO of this invention is described in WO 86/03520.

The method of Miyake et al. comprises deactivating any proteolytic enzymes by treating the crude EPO preparations with phenol p-aminosalicylate. Such proteases can also be deactivated by other means such as by heating. The purification steps described by Miyake et al. include ethanol precipitation, DEAE-agarose fractionation, sulfopropyl-Sephadex chromatography, gel filtration and hydroxylapatite chromatography. The "purified" EPO compositions obtained thereby reportedly have a specific EPO activity of at least about 50,000, preferably at least about to 80,000 IU, per absorbance unit at 280 nm. (The prior art thus considered "pure" EPO as having a specified activity of about 80,000 IU).

We found the "purified" Miyake EPO composition to be non-homogeneous. By further treating this composition by reverse phase high performance liquid chromatography (R-P HPLC) we obtained a homogeneous EPO having a molecular weight of about 34,000 daltons when analyzed by sodium dodecyl sulfate polyacrylamide gel electrophoresis (SDS-PAGE).

The process of the invention for purifying erythropoietin of natural origin therefore comprises the following steps:

a) deactivation of any proteolytic enzymes;
b) ethanol precipitation;
c) DEAE-agarose fractionation;
d) sulfopropyl-Sephadex® chromatography;
e) gel filtration;
f) hydroxylapatite chromatography;

and is characterized by further treating this composition by

g) reverse phase high performance liquid chromatography; and

h) eluting therefrom homogeneous erythropoietin which moves as a single peak on reverse phase high performance liquid chromatography; exhibits a molecular weight of about 34,000 daltons on SDS-PAGE; and possesses a specific activity of about 160,000 IU per absorbance unit at 280 nanometers.

A preferred reverse phase HPLC column for use in this invention is a column having four-carbon (butyl) chain groups attached thereto. A commercial example is the C-4 Vydac® column available from The Nest Group, Southboro, MA. Other reverse columns with different carbon chain groups, such as eight-carbon or eighteen-carbon, may also be used. A preferred eluant consists of a 0 to 95% acetonitrile gradient in 0.01 to 1.0%, preferably 0.1% trifluoroacetic acid, over a period of about 100 minutes. Other eluants can of course also be used.

When purified in accordance with the present invention, EPO compositions having a specific activity of at least 160,000 IU per absorbance unit at 280 nm are obtained.

An "absorbance unit," as used herein, is approximately 1 mg protein per ml.

The amino acid sequence of an EPO protein derived from a human source, including its secretory leader sequence is illustrated in Table I. The mature EPO protein begins with the "Ala" residue identified by the arabic number "1". The secretory leader sequence is the polypeptide sequence preceding the mature EPO protein beginning with the "MET" residue identified by the numeral "-27". This DNA sequence can be expressed in a cell capable of processing the EPO protein to eliminate the leader sequence and secrete the mature protein into the media.

In clinical uses, e.g., in the treatment of various anemias, the amount of EPO will, of course, depend upon the severity of the condition being treated, the route of administration chosen, and the specific activity of the EPO, and ultimately will be decided by the attending physician.

EPO may be administered by any route appropriate to the condition being treated. Preferably, the EPO is injected into the bloodstream.

The formulations of the present invention comprise an active EPO protein, as above described, together with one or more pharmaceutically acceptable carriers therefor and optionally other therapeutic ingredients. The carrier(s) must be "acceptable" in the sense of being compatible with the other ingredients of the formulation and not deleterious to the recipient thereof.

Formulations suitable for parenteral administration conveniently comprise a sterile parenterally acceptable vehicle, e.g., water containing a therapeutically effective amount of EPO. Solutions are preferably isotonic.

The following example illustrates the preparation of the product of the present invention.


EXAMPLE

Crude erythropoietin preparations, derived from patients with aplastic anemia, were concentrated by dialysis. Proteolytic enzymes were deactivated by heat treatment at 80°C for 5 minutes. The crude preparation concentrates were then purified by the above method described by Miyake et al.


A. Ethanol Precipitation

Batches containing about 100,000 IU of EPO activity at concentrations of about 50 to 100 IU per absorbance unit at 280 nm were diluted to 50 ml with phosphate buffered solution (PBS) at 4°C. 12.5 ml of 10 M LiCl were added. Absolute ethanol (62.5 ml) at 4° was added slowly with stirring, which was continued for 30 min. after the addition was complete. After the flocculent precipitate had been allowed to settle for 10 min. it was removed by centrifugation at 21,000 x g for 10 min at -15°. The pellet was washed three times with 10 ml of 50% ethanol, 1 m LiCl and the supernatants were pooled. The washed precipitate was dissolved in 20 ml of PBS, yielding a turbid solution (50% precipitate.)

Sixty-seven milliliters of absolute ethanol were added slowly to the combined supernatants: stirring was continued for 30 min. and settling for 15 min. The precipitate was collected as before and washed twice with 10 ml of 65% ethanol, 0.7 M LiCl and the supernatants were pooled. The washed precipitate was dissolved in 20 ml of PBS (65% precipitate).

To the pooled supernatants, 96 ml of ethanol were added slowly and stirring was continued for 30 min. after which the precipitate was allowed to settle for 14 h at 4°. The precipitate was washed twice with 10 ml of 75% ethanol, 0.5 M LiCl, the supernatants were pooled, and the precipitate was dissolved in 20 ml of PBS (75% precipitate).

The combined supernatant was brought to 90% ethanol by addition of 540 ml of absolute alcohol, stirred for 30 min. and stored at -20° for 48 hrs. before the precipitate was collected, dissolved in 50 ml of cold water and immediately frozen.

B. DEAE - Agarose Fractionation

The solution, in water, of a 90% ethanol precipitate was concentrated to about 5 ml of an Amicon UM-10 ultrafilter, then brought to 25 ml with 0.01 M Tris, pH 7.0, and a 50-ul aliquot was removed. The DEAE-agarose, 100 to 200 mesh, was degassed under reduced pressure, suspended in 0.01 M Tris, pH 7.0, and packed into a column 9.2 x 2.5 cm in diameter (bed volume, 45 ml). The gel was washed with 1.5 liters of 0.01 M Tris, pH 6.9; the ratio of absorbance units added to bed volume (ml) was 6.65. The sample was added to the column over a period of 40 min. and 150-drop fractions were collected. The column was washed with 211 ml of 0.01 M Tris, pH 7, and then eluted with the following buffers: 366 ml of 0.01 M Tris, pH 7.0; 5mM $CaCl_2$, 270 ml of 0.01 Tris, pH 7.0; 17 mM $CaCl_2$; 194 ml of 0.01 M Tris, pH 7.0;3 mM $CaCl_2$; and 65 ml of 0.1 M $CaCl_2$.

From this point on in the fractionation calcium was added to all buffers except those used with hydroxylapatite columns because there were inconsistent results and appreciable losses of activity when buffers without calcium were used. For the next step in purification, eluates from DEAE agarose columns were selected that had significant quantities of EPO activity.

C. Sulfopropyl-Sephadex® Chromatography

The eluates (17 mM $CaCl_2$) from DEAE-agarose columns were desalted and concentrated on a UM-10 ultafilter and then dialyzed against 2 liters of 5 mM $CaCl_2$, pH 7.5 overnight. In the sample run described below, 30 ml of dialyzed solution were brought to pH 4.50 by dropwise addition of 0.1 M HCl: the small amount of precipitate formed was removed by centrifugation and washed with 5 ml of 5 mM $CaCl_2$, pH 4.5. The wash, pooled with the supernatant, was applied to a sulfopropyl-Sephadex® column (15.0 x 2.5 cm in diameter, bed volume, 78.3 ml) which had been equilibrated with 5 mM $CaCl_2$, pH 4.50. The absorbance units to bed volume (ml) ratio was 2.47. A low value for this ratio is preferred for optimal fractionation on sulfopropyl-Sephadex®: for example, if the absorbance unit to bed volume ratio was greater than 10, almost all of the activity was found in the effluent fraction. The following buffers were used in developing the column. Input was: 5 mM calcium acetate, pH 4.50, specific conductivity = 1.075 $\mu$mho cm$^{-1}$. Eluting buffers were: 7.5 mM calcium acetate, pH 4.70, specific conductivity = 2,100 $\mu$mho cm$^{-1}$: 15 mM calcium acetate, pH 5.25, specific conductivity = 2,100 $\mu$mho cm$^{-1}$: 15 mM calcium acetate, 0.01 m Tris, pH 7.24, specific conductivity = 11,500 $\mu$mho cm$^{-1}$. The column was run at 0.4 ml/min at 4°, and 200-drop fractions were collected. After a reading was taken at 280 nm and the appropriate pools were made, the solutions were neutralized (within 1 h after elution) and aliquots were removed for assay and stored at -20°.

D. Gel Filtration

The 12.5 and 15 mM calcium acetate eluates from the sulfopropyl-Sephadex® column separations were run in two separate batches on the same gel column. The pools were concentrated on Amicon UM-2 ultrafilters to about 5 ml and equilibrated with 10 mM $CaCl_2$, 10 mM Tris, pH 6.87, before application to the column. The Sephadex® G-100 gel was degassed under reduced pressure and equilibrated with the same buffer before the column was poured. The column (100 x 2.5 cm diameter) was calibrated with markers of known molecular size before being used for the erythropoietin fractions. The void volume was 135 ml; bovine serum albumin monomer eluted at 224 ml. ovalbumin at 258 ml, and cytochrome at 368 ml. The sample was added to the bottom of the column, as was the buffer which was passed through the column at 21 to 22 ml by means of Mariotte bottle with a 42 cm hydrostatic head. Each fraction collected was 4.1 ml (120 drop), and pools were made. The pools were concentrated by ultrafiltration and aliquots were assayed.

E. Hydroxylapatite Chromatography

Hydroxylapatite was packed under unit gravity into a column (6.1 x 1.5 cum diameter) and washed with 500 ml of water and then with 400 ml of 0.5 mM phosphate buffer, pH 7.1, conductivity = 69 $\mu$mho cm$^{-1}$ (Buffer I), by use of a peristaltic pump which maintained the flow at 0.3 ml/min. After the buffer wash, the length of the column was 3.4 cm and the bed volume was 6.0 ml. the input sample was concentrated and desalted on an Amicon DM-5 ultrafilter by adding water to the concentrate and the wash of the filter was centrifuged at 6,000 x g for 20 min. at 4°. The small insoluble pellet was washed once with 0.5 mM phosphate, pH 7.1, and the wash was added to the supernatant. An aliquot for assay was removed and the remainder (22 ml) was added to the column. The ratio of absorbance units added to bed volume (ml) was 1.82. The input buffer was pumped through the column until the effluent A was less than 0.005 (149 ml) and

5

the following elution schedule was carried out: Buffer II, 1 mM phosphate (pH 7.1, specific conductivity 131 = $\mu$mho cm $^{-1}$, 150 ml (Fraction II)); Buffer III, 2 mM phosphate (pH 6.9, specific conductivity = 270 $\mu$mho cm$^{-1}$, 220 ml (fractions IIIA and IIIB)); Buffer IV, 3 mM phosphate (pH 6.9, specific conductivity = 402 umho cm$^{-1}$, 84 ml (Fraction IV)); Buffer V, 0.1 M phosphate (pH 6.8, specific conductivity = 9.6 $\mu$mho cm$^{-1}$, 134 ml (Fraction V)).

Fractions containing EPO were concentrated by means of Amicon DM-5 ultrafilter, an aliquot assayed and the concentrate stored frozen. The assay indicated a specific EPO activity of 83,000 IU per absorbance unit at 280 nm.

## F. Reverse Phase HPLC

When analyzed by sodium dodecyl sulfate polyacrylamide gel electrophoresis (SDS PAGE) according to Laemmli, U.K., Nature, 15:Vol. 22, No. 259 pp. 680-685 (1970) the material from the hydroxylapatite column revealed several polypeptide components ranging from approximately 70,000 MW to 30,000 MW with the major component at about 34,000 MW. This preparation of EPO which was obtained in volumes of up to 10 ml in 10 mM phosphate buffer pH 7.0 was subjected to R-P HPLC as described below.

The EPO preparation was concentrated 10-fold by partial lyophilization. Approximately 200 microliters of this concentrated material was injected onto a R-P HPLC column, the type with four-carbon groups attached (a commercial example is a C-4 Vydac), (25 x 0.45 cm, the Separations Group) and fractionated by a R-P HPLC using the gradient conditions described in Table 2.

## TABLE 2

Pump A    0.1% Trifluoracetic Acid (TFA) in water

Pump B    0.1% TFA in 95% Acetonitrile; 5% $H_2O$

| Gradient Time (Min.) | % B | Duration |
|---|---|---|
| 0 | 0 | 2 |
| 2 | 25 | 3 |
| 5 | 100 | 75 |
| 90 | 0 | 3 |

Flow 1 ml/min.

EPO is quantified by either the 3H-thymidine assay (Krystal, Exp. Hematol. 11:649-60 (1983)) or CFU-E assay (Bersch et al., In vitro Aspects of Erythropoiesis, M. J. Murphy (Ed.), New York: Springer-Verloz (1978)).

Protein peaks were detected by UV absorption at 280 nm. A typical elution profile of this fractionation process is shown in Fig. 1. The homogenous nature of EPO was confirmed by SDS PAGE and N-terminal amino acid sequence analysis of the various peaks observed after R-P HPLC. The peak that is underlined in Fig. 1 elutes coincidentally with a reading of 53% on the gradient maker (Beckman Instruments, model 421). This material runs as a single band of about 34,000 MW using SDS PAGE and yields a single amino terminal sequence of: Ala, Pro, Pro, Arg, Leu, Ile, Cys - as has been previously reported for human EPO. Only this R-P HPLC fraction of about 34,000 MW showed any significant biological activity in vitro. The EPO protein eluted by R-PHPLC is about twice as pure as the material eluted from the hydroxylapatite column (STEP E).

## Claims

**Claims for the following Contracting States : BE, CH, DE, FR, GB, IT, LI, LU, NL, SE**

**1.** A process for purifying erythropoietin of natural origin comprising the following steps:
   a) deactivation of any proteolytic enzymes;
   b) ethanol precipitation;
   c) DEAE-agarose fractionation;
   d) sulfopropyl-Sephadex® chromatography;

EP 0 209 539 B1

e) gel filtration;
f) hydroxylapatite chromatography;
characterized by further treating this composition by
g) reverse phase high performance liquid chromatography; and
h) eluting therefrom homogeneous erythropoietin which moves as a single peak on reverse phase high performance liquid chromatography; exhibits a molecular weight of about 34,000 daltons on SDS-PAGE; and possesses a specific activity of about 160,000 IU per absorbance unit at 280 nanometers.

2. Homogeneous erythropoietin, characterized by:
a) movement as a single peak on a reverse phase-HPLC;
b) a molecular weight of about 34,000 daltons on SDS-PAGE; and
c) a specific activity of about 160,000 IU per absorbance at 280 nm.

3. A pharmaceutical composition for the treatment of anemia comprising a therapeutically effective amount of the homogeneous erythropoietin of claim 2 in a pharmaceutically acceptable vehicle.

**Claims for the following Contracting State : AT**

1. A process for purifying erythropoietin of natural origin comprising the following steps:
a) deactivation of any proteolytic enzymes;
b) ethanol precipitation;
c) DEAE-agarose fractionation;
d) sulfopropyl-Sephadex® chromatography;
e) gel filtration;
f) hydroxylapatite chromatography;
characterized by further treating this composition by
g) reverse phase high performance liquid chromatography; and
h) eluting therefrom homogeneous erythropoietin which moves as a single peak on reverse phase high performance liquid chromatography; exhibits a molecular weight of about 34,000 daltons on SDS-PAGE; and possesses a specific activity of about 160,000 IU per absorbance unit at 280 nanometers.

2. Homogeneous erythropoietin, characterized by:
a) movement as a single peak on a reverse phase-HPLC;
b) a molecular weight of about 34,000 daltons on SDS-PAGE; and
c) a specific activity of about 160,000 IU per absorbance at 280 nm.

**Revendications**

**Revendications pour les Etats contractants suivants : BE, CH, DE, FR, GB, IT, LI, LU, NL, SE**

1. Un procédé pour purifier l'érythropoïétine d'origine naturelle comprenant les étapes suivantes :
a) désactivation de toute enzyme protéolytique ;
b) précipitation par l'éthanol ;
c) fractionnement avec du DEAE-agarose ;
d) chromatographie avec du sulfopropyl-Sephadex® ;
e) filtration sur gel ;
f) chromatographie avec de l'hydroxy-apatite ; caractérisé de plus par le traitement de cette composition par
g) chromatographie liquide à haute performance en phase inverse ; et
h) élution de l'érythropoïétine homogène qui migre en un pic unique en chromatographie liquide à haute performance en phase inverse ; présente un poids moléculaire d'environ 34 000 daltons en électrophorèse sur gel de dodécylsulfate de sodium et de polyacrylamide ; et possède une activité spécifique d'environ 160 000 UI par unité d'absorbance à 280 nanomètres.

2. Erythropoïétine homogène, caractérisée par :
a) la migration en un pic unique en chromatographie liquide à haute performance en phase inverse ;

7

b) un poids moléculaire d'environ 34 000 daltons en électrophorèse sur gel de dodécylsulfate de sodium et de polyacrylamide ; et

c) une activité spécifique d'environ 160 000 UI par unité d'absorbance à 280 nm.

**3.** Une composition pharmaceutique pour le traitement de l'anémie comprenant une quantité thérapeutique efficace de l'érythropoïétine homogène de la revendication 2 dans un véhicule pharmaceutiquement acceptable.

**Revendications pour l'Etat contractant suivant : AT**

**1.** Procédé pour purifier l'érythropoïétine d'origine naturelle comprenant les étapes suivantes :
a) désactivation de toute enzyme protéolytique ;
b) précipitation par l'éthanol ;
c) fractionnement avec du DEAE-agarose ;
d) chromatographie avec du sulfopropyl-Sephadex® ;
e) filtration sur gel ;
f) chromatographie avec de l'hydroxy-apatite ; caractérisé de plus par le traitement de cette composition par
g) chromatographie liquide à haute performance en phase inverse ; et
h) élution de l'érythropoïétine homogène qui migre en un pic unique en chromatographie liquide à haute performance en phase inverse ; présente un poids moléculaire d'environ 34 000 daltons en électrophorèse sur gel de dodécylsulfate de sodium et de polyacrylamide ; et possède une activité spécifique d'environ 160 000 UI par unité d'absorbance à 280 nanomètres.

**2.** Erythropoïétine homogène, caractérisée par :
a) la migration en un pic unique en chromatographie liquide à haute performance en phase inverse ;
b) un poids moléculaire d'environ 34 000 daltons en électrophorèse sur gel de dodécylsulfate de sodium et de polyacrylamide ; et
c) une activité spécifique d'environ 160 000 UI par unité d'absorbance à 280 nm.

**Patentansprüche**

**Patentansprüche für folgende Vertragsstaaten : BE, CH, DE, FR, GB, IT, LI, LU, NL, SE**

**1.** Verfahren zur Reinigung von Erythropoietin natürlichen Ursprungs, umfassend die folgenden Schritte:
a) Inaktivierung von proteolytischen Enzymen,
b) Ethanolfällung,
c) DEAE-Agarose-Fraktionierung,
d) Sulfopropyl-Sephadex® Chromatographie,
e) Gelfiltration,
f) Hydroxylapatit-Chromatographie,
**gekennzeichnet durch** weitere Behandlung dieser Zusammensetzung mit
g) Reverse Phase-Hochauflösungs-Flüssigkeitschromatographie und
h) Eluieren von homogenem Erythropoietin daraus, das als einzelner Peak bei Reverse Phase-Hochauflösungs-Flüssigkeitschromatographie wandert, ein Molekulargewicht von etwa 34.000 Dalton bei SDS-PAGE aufweist und eine spezifische Aktivität von etwa 160.000 IU pro Absorptionseinheit bei 280 Nanometern besitzt.

**2.** Homogenes Erythropoietin, **gekennzeichnet durch**:
a) Wanderung als einzelner Peak bei einer Reverse Phase-HPLC,
b) ein Molekulargewicht von etwa 34.000 Dalton bei SDS-PAGE und
c) eine spezifische Aktivität von etwa 160.000 IU pro Absorptionseinheit bei 280 Nanometern.

**3.** Pharmazeutische Zusammensetzung zur Behandlung von Anämie, umfassend eine therapeutisch wirksame Menge des homogenen Erythropoietins nach Anspruch 2 in einem pharmazeutisch verträglichen Träger.

**Patentansprüche für folgenden Vertragsstaat : AT**

8

**1.** Verfahren zur Reinigung von Erythropoietin natürlichen Ursprungs, umfassend die folgenden Schritte:

a) Inaktivierung von proteolytischen Enzymen,

b) Ethanolfällung,

c) DEAE-Agarose-Fraktionierung,

d) Sulfopropyl-Sephadex® Chromatographie,

e) Gelfiltration,

f) Hydroxylapatit-Chromatographie,

**gekennzeichnet durch** weitere Behandlung dieser Zusammensetzung mit

g) Reverse Phase-Hochauflösungs-Flüssigkeitschromatographie und

h) Eluieren von homogenem Erythropoietin daraus, das als einzelner Peak bei Reverse Phase-Hochauflösungs-Flüssigkeitschromatographie wandert, ein Molekulargewicht von etwa 34.000 Dalton bei SDS-PAGE aufweist und eine spezifische Aktivität von etwa 160.000 IU pro Absorptionseinheit bei 280 Nanometern besitzt.

**2.** Homogenes Erythropoietin, **gekennzeichnet durch**:

a) Wanderung als einzelner Peak bei einer Reverse Phase-HPLC,

b) ein Molekulargewicht von etwa 34.000 Dalton bei SDS-PAGE und

c) eine spezifische Aktivität von etwa 160.000 IU pro Absorptionseinheit bei 280 Nanometern.

FIG.1

TABLE 1

|  |  |  |  |  |  |  |  |  |  |  |  |  | -27 MET | GLY | VAL | HIS | GLU | CYS | PRO |
|---|---|---|---|---|---|---|---|---|---|---|---|---|---|---|---|---|---|---|---|
| ALA | TRP | LEU | TRP | LEU | LEU | LEU | SER | LEU | LEU | SER | LEU | PRO | LEU | GLY | LEU | PRO | VAL | LEU | GLY |
| 1 Ala | Pro | Pro | Arg | Leu | Ile | Cys SH | Asp | Ser | 10 Arg | Val | Leu | Glu | Arg | Tyr | Leu | Leu | Glu | Ala | 20 Lys |
| Glu | Ala | Glu | Asn | Ile | Thr | Thr | Gly | Cys SH | 30 Ala | Glu | His | Cys | Ser | Leu | Asn | Glu | Asn | Ile | 50 Thr |
| Val | Pro | Asp | Thr | Lys | Val | Asn | Phe | Tyr | 50 Ala | Trp | Lys | Arg | Met | Glu | Val | Gly | Gln | Gln | 60 Ala |

EP 0 209 539 B1

3A

TABLE 1 (CONTI'D)

|     |     |     |     |     |     |     |     |     |  70 |     |     |     |     |     |     |     |     |     |  80 |
|-----|-----|-----|-----|-----|-----|-----|-----|-----|-----|-----|-----|-----|-----|-----|-----|-----|-----|-----|-----|
| Val | Glu | Val | Trp | Gln | Gly | Leu | Ala | Leu | Leu | Ser | Glu | Ala | Val | Leu | Arg | Gly | Gln | Ala | Leu |
| Leu | Val | Asn | Ser | Ser | Gln | Pro | Trp | Glu | Pro<br>90 | Leu | Gln | Leu | His | Val | Asp | Lys | Ala | Val | Ser<br>100 |
| Gly | Leu | Arg | Ser | Leu | Thr | Thr | Leu | Leu | Arg<br>110 | Ala | Leu | Gly | Ala | Gln | Lys | Glu | Ala | Ile | Ser<br>120 |
| Pro | Pro | Asp | Ala | Ala | Ser | Ala | Ala | Pro | Leu<br>130 | Arg | Thr | Ile | Thr | Ala | Asp | Thr | Phe | Arg | Lys<br>140 |
| Leu | Phe | Arg | Val | Tyr | Ser | Asn | Phe | Leu | Arg<br>150 | Gly | Lys | Leu | Lys | Leu | Tyr | Thr | Gly | Glu | Ala<br>160 |

Cys Arg Thr Gly Asp Arg
 SH                  166